# EUROPEAN PATENT APPLICATION

(11) **EP 4 840 426 A2**
(43) Date of publication of application: **18.11.2026**
(21) Application number: 26178549.7
(22) Date of filing: 13.05.2026
(51) Int. Cl.: A61F 5/058, B33Y 80/00

(54) **HEAD ORTHOTIC WITH HINGE ASSEMBLY AND CLASP**

(30) Priority: 15.05.2025 US 202519209687
(71) Applicant: Hanger, Inc., Austin TX 78758 (US)
(72) Inventor: DIAS, Antonio, Arizona, 85040 (US); FLORES, Aaron, Austin, 78746 (US); KLEPPE, Ryan, Austin, 78758 (US); MIETH, Justin, Austin, 78758 (US); STEVENS, Phil, North Salt Lake, 84054 (US); TOMPKINS, Michael E., Austin, 78758 (US)
(74) Representative: Potter Clarkson

(57) **Abstract**

A system for manufacturing a cranial remodeling orthosis (CRO) includes a scan device, a 3D printer, and processing circuitry. The scan device is configured to obtain scan data of a patient's head. The processing circuitry is configured to obtain the scan data of the patient's head. The processing circuitry is configured to use computer assisted design to generate a print file of the CRO based on the scan data, where the CRO includes at least one integrated closure mechanism. The 3D printer is configured to perform additive manufacturing to produce the CRO using the print file of the CRO.

## Description

### CROSS-REFERENCE TO RELATED PATENT APPLICATION

This application claims priority to and the benefit of U.S. Application No. 19/209,687, filed May 15, 2025 under the Paris Convention. This application is related to U.S. Application No. 19/209,459, filed May 15, 2025 and U.S. Application No. 19/109,473, filed March 6, 2025, which is a PCT National Phase conversion of International Application No. PCT/US2023/032048, filed September 6, 2023, which claims priority to U.S. Provisional Patent Application No. 63/404,343, filed September 7, 2022; this application is also related to U.S. Application No. 18/594,940, filed March 4, 2024, which is a continuation of U.S. Application No. 17/373,147, filed July 12, 2021, which is a continuation-in-part of U.S. Application No. 16/050,849, filed July 31, 2018, which is a continuation of International Application No. PCT/US2017/015981, filed February 1, 2017, which claims priority to U.S. Provisional Patent Application No. 62/290,254, filed February 2, 2016; this application is also related to U.S. Application No. 18/594,873, filed March 4, 2024, which is a continuation of U.S. Application No. 17/373,147, filed July 12, 2021, which is a continuation-in-part of U.S. Application No. 16/050,849, filed July 31, 2018, which is a continuation of International Application No. PCT/US2017/015981, filed February 1, 2017, which claims priority to U.S. Provisional Patent Application No. 62/290,254, filed February 2, 2016; this application is also related to a U.S. Application No. 17/547,507, filed December 10, 2021, which claims priority to U.S. Provisional Patent Application No. 63/124,230, filed December 11, 2020; and this application is also related to continuation-in-part of U.S. Application No. 18/910,527, filed October 9, 2024, which is a continuation of U.S. Application No. 18/852,840, filed September 30, 2024, which is a PCT National Phase conversion of International Application No. PCT/US2023/036863, filed November 6, 2023, which claims priority to U.S. Provisional Patent Application No. 63/423,311, filed November 7, 2022, all of which are incorporated herein by reference in their entireties and for all purposes.

### BACKGROUND

The present disclosure relates generally to prosthetics and orthotics. More particularly, the present disclosure relates to additive manufacturing of a cranial orthotic.

### SUMMARY

One implementation of the present disclosure is a method, according to some embodiments. In some embodiments, the method includes obtaining scan data of a patient's head. In some embodiments, the method includes using computer assisted design to generate a print file of a cranial remodeling orthosis (CRO) based on the scan data, where the CRO includes at least one integrated closure mechanism. In some embodiments, the method includes performing additive manufacturing by a 3D printer to produce the CRO using the print file of the CRO.

In some embodiments, the CRO includes an outer shell configured to receive a patient interfacing insert, where at least one of the outer shell or the patient interfacing insert are manufactured by the 3D printer. In some embodiments, the at least one integrated closure mechanism includes a hinge. In some embodiments, the hinge is configured to facilitate relative rotation of the first section and the second section to transition the CRO between an open configuration and a closed configuration.

In some embodiments, using the computer assisted design to generate the print file includes modifying a position, thickness, and trim area of the print file such that the CRO produced using the print file is configured to provide therapy to reshape the patient's head over time. In some embodiments, using the computer assisted design to generate the print file includes drawing lines and shapes onto the scan data of the patient's head and generating the print file based on the lines and shapes. In some embodiments, the CRO is a helmet configured to be worn on the patient's head.

Another implementation of the present disclosure is a system for manufacturing a cranial remodeling orthosis (CRO), according to some embodiments. In some embodiments, the system includes a scan device, processing circuitry, and a 3D printer. In some embodiments, the scan device is configured to obtain scan data of a patient's head. In some embodiments, the processing circuitry is configured to obtain the scan data of the patient's head. In some embodiments, the processing circuitry is configured to use computer assisted design to generate a print file of the CRO based on the scan data, where the CRO includes at least one integrated closure mechanism. In some embodiments, the 3D printer is configured to perform additive manufacturing to produce the CRO using the print file of the CRO.

In some embodiments, the CRO includes an outer shell configured to receive a patient interfacing insert, where at least one of the outer shell or the patient interfacing insert are manufactured by the 3D printer. In some embodiments, the outer shell includes a first section and a second section. In some embodiments, the at least one integrated closure mechanism includes a hinge. In some embodiments, the hinge is configured to facilitate relative rotation of the first section and the second section to transition the CRO between an open configuration and a closed configuration.

In some embodiments, using the computer assisted design to generate the print file includes modifying a position, thickness, and trim area of the print file such that the CRO produced using the print file is configured to provide therapy to reshape the patient's head over time. In some embodiments, using the computer assisted design to generate the print file includes drawing lines and shapes onto the scan data of the patient's head and generating the print file based on the lines and shapes. In some embodiments, the CRO is a helmet configured to be worn on the patient's head.

Another implementation of the present disclosure is a cranial remodeling orthosis (CRO) configured to be worn on a patient's head, according to some embodiments. In some embodiments, the CRO includes an outer shell and internal padding. In some embodiments, the outer shell includes at least one integrated closure mechanism. In some embodiments, the internal padding is configured to couple with the outer shell along an inwards facing surface of the outer shell. In some embodiments, the CRO is configured to adjust a shape of a skull of the patient over time to increase symmetry of the patient's head. In some embodiments, at least one of the outer shell or the internal padding are manufactured by additive manufacturing.

In some embodiments, the outer shell includes a first section and a second section. In some embodiments, the at least one integrated closure mechanism includes a hinge. In some embodiments, the hinge is configured to facilitate relative rotation of the first section and the second section to transition the CRO between an open configuration and a closed configuration.

This summary is illustrative only and is not intended to be in any way limiting. Other aspects, inventive features, and advantages of the devices or processes described herein will become apparent in the detailed description set forth herein, taken in conjunction with the accompanying figures, wherein like reference numerals refer to like elements.

### BRIEF DESCRIPTION OF THE DRAWINGS

The disclosure will become more fully understood from the following detailed description, taken in conjunction with the accompanying figures, wherein like reference numerals refer to like elements, in which:
FIG. 1 is a front view of a pediatric orthotic device, according to some embodiments.
FIG. 2 is a side view of the pediatric orthotic device of FIG. 1, according to some embodiments.
FIG. 3 is a top view of the pediatric orthotic device of FIG. 1, according to some embodiments.
FIG. 4 is a perspective view of the pediatric orthotic device of FIG. 1 including a clasp mechanism in a closed configuration, according to some embodiments.
FIG. 5 is a perspective view of the pediatric orthotic device of FIG. 1 including a hinge mechanism in a closed configuration, according to some embodiments.
FIG. 6 is a front view of the pediatric orthotic device of FIG. 1 including the clasp mechanism of FIG. 4 and the hinge mechanism of FIG. 5 in the closed configuration, according to some embodiments.
FIG. 7 is a top view of the pediatric orthotic device of FIG. 1 including the clasp mechanism of FIG. 4 and the hinge mechanism of FIG. 5 in an open configuration, according to some embodiments.
FIG. 8 is a side view of the pediatric orthotic device of FIG. 1 including the hinge mechanism of FIG. 5 in an open configuration, according to some embodiments.
FIG. 9 is a side view of the pediatric orthotic device of FIG. 1 including the clasp mechanism of FIG. 4 in an open configuration, according to some embodiments.
FIG. 10 is an illustration of the clasp mechanism of FIG. 4, according to some embodiments.
FIG. 11 is an illustration of the hinge mechanism of FIG. 5, according to some embodiments.
FIG. 12A is a front view of an internal pad of the pediatric orthotic device of FIGS. 1-9, according to some embodiments.
FIG. 12B is a side view of the internal pad of FIG. 12A, according to some embodiments.
FIG. 13 is a flow diagram of a process for manufacturing the pediatric orthotic device of FIGS. 1-12B, according to some embodiments.
FIG. 14 is a system for additive manufacturing that can be used to manufacture the pediatric orthotic device of FIGS. 1-12B, according to some embodiments.
FIG. 15 is a diagram of a recommendation system including a recommendation tool configured to use scan data of a patient's head to recommend one or more cranial remodeling orthosis components, according to some embodiments.
FIG. 16 is a top view showing a comparison between scan data and a target head shape across different quadrants, according to some embodiments.
FIG. 17 is a side view showing a comparison between scan data and a target head shape across different quadrants, according to some embodiments.
FIG. 18 is a perspective view of a pad for the pediatric orthotic device of FIGS. 1-9, according to some embodiments.
FIG. 19 is a table illustrating information that may be stored in a pads database for the recommendation tool of FIG. 15, according to some embodiments.
FIG. 20 is a table illustrating information that may be stored in a shell database for the recommendation tool of FIG. 15, according to some embodiments.
FIG. 21 is a flow diagram of a process for using scan data of a patient's head to recommend one or more cranial remodeling orthosis components, according to some embodiments.

### DETAILED DESCRIPTION

Before turning to the FIGURES, which illustrate the exemplary embodiments in detail, it should be understood that the present application is not limited to the details or methodology set forth in the description or illustrated in the FIGURES. It should also be understood that the terminology is for the purpose of description only and should not be regarded as limiting.

### Overview

Referring generally to the FIGURES, additive manufacturing can be used to produce a pediatric orthotic device such as a cranial remodeling orthosis that is configured to non-invasively remodel a patient's skull. The pediatric orthotic device is designed such that internal padding is used in conjunction with an exterior shell to create a custom design for each patient. The internal padding abuts, directly contacts, engages, etc., areas of a patient's head where growth is to be discouraged or minimized, and may be spaced apart from areas of the patient's head where growth is to be encouraged. In this way, the pediatric orthotic device can correct asymmetries of the patient's skull or head without providing any clamping force to the patient's head. Furthermore, the pediatric orthotic device can utilize a clasp mechanism and a hinge mechanism to secure the pediatric orthotic device on the patient's head. Advantageously, the clasp mechanism and the hinge mechanism can be configured to create a sufficiently wide opening in the pediatric orthotic device such that the pediatric orthotic device can be placed on or removed from the patient's head without providing any clamping force, even if the patient's head is not still. As another benefit, the clasp mechanism and the hinge mechanism provide a clearer visual indication of whether the pediatric orthotic device is secured to the patient's head or not compared to other mechanisms (e.g., Velcro). The exterior shell and the internal padding can be manufactured using a 3d printer in a layer by layer process, according to some embodiments.

Advantageously, the orthotic device may adjust a shape of the patient's skull over time to increase symmetry of the patient's head. The orthotic device can be tailored in design to prevent growth in any unintended directions and to allow growth in any directions that will increase the symmetry of the patient's head over time. The design of the orthotic device can be modified and adjusted to provide proper distribution of forces across the skull such that the patient's head will become more symmetrical with use of the device.

In some embodiments, the pediatric orthotic device can be created using an entirety of additively manufactured components, an entirety of standard or pre-manufactured parts or components, or a combination of additively manufactured and standard or pre-manufactured parts or components. The pediatric orthotic device may be a custom prescriptive device with standard of pre-manufactured parts. The exterior shell can be created from a library of shapes that utilize a pegboard design of receivers, according to some embodiments. The internal padding can also be created from a library of shapes and can be inserted into the receivers on an inwards facing surface of the exterior shell, according to some embodiments. In some embodiments, the internal padding component is manufactured by an injection molding process or additive manufacturing. The process described herein of producing the device allows for same day point of service as a patient's visit to eliminate manufacturing delays due to heating foam pads, gluing the foam to the shell, etc.

In some embodiments, the pediatric orthotic device has an entirety of its components produced via additive manufacturing (e.g., entirely 3d printed components). A method of creating the device includes taking a 3d scan of the patient's head to capture anatomical features and landmarks for reference in the design process, according to some embodiments. In some embodiments, the 3d scan of the patient's head generates a scan file. The scan file is then converted to a design file (e.g., computer assisted design (CAD) file and/or computer assisted manufacturing (CAM) file), according to some embodiments. In some embodiments, build-ups, reductions, smoothing, and other modifications are made to the CAD/CAM file to generate a 3d model of the device based on the anatomy and requirements of the patient.

In some embodiments, the CAD/CAM file is then used as the basis for the device design, where offsets are created for each component of the device and trimlines are drawn for creating the appropriate device shape. The individual components of the CAD/CAM file (e.g., the exterior shell and internal padding) are then exported separately and uploaded to an additive manufacturing device, such as a 3d printer, where the components are created in a layer by layer process, according to some embodiments. Following the manufacturing process, the components are then post-processed to remove any excess material or undesired aspects, according to some embodiments. Once post-processed, the components are then assembled to create the device in the final assembly stage, according to some embodiments. The end result is a pediatric orthotic device with an exterior shell and internal padding that conforms to the anatomy of the patient's head and can perform the intended cranial reshaping functionality, according to some embodiments.

In some embodiments, a method of creating the pediatric orthotic device includes using an algorithm that identifies a combination of standard or pre-manufactured parts or components, including the exterior shell and the internal padding to result in a custom prescriptive device. Inputs into the algorithm can be the scan file generated from the scan of the patient's head or a list of measurements provided by a user (e.g., a health care provider). Once identified as standard or pre-manufactured parts or components, the exterior shell and the internal padding can be assembled to create a custom device for the patient, according to some embodiments. The end result is a pediatric orthotic device with an exterior shell and internal padding that conforms to the anatomy of the patient's head and can perform the intended cranial reshaping functionality, according to some embodiments.

The material composition of the exterior shell of the pediatric orthotic device consists entirely of a versatile thermoplastic, according to some embodiments. The material composition of the internal padding of the device consists entirely of foam or an equivalent padding material, according to some embodiments. The material composition of the exterior shell and the inner padding of the device is such that the device is lightweight for increased patient comfortability, according to some embodiments. The material composition of the components of the device allows for minor adjustments to be made to the device's overall shape using targeted heat following additive manufacturing.

In some embodiments, the prosthetic, orthotic, protective device, etc., as described herein is manufactured using any of the techniques as described in U.S. Patent No.: 10,766,246 B2, filed December 15, 2014, the entire disclosure of which is incorporated by reference herein.

### Pediatric Orthotic Device

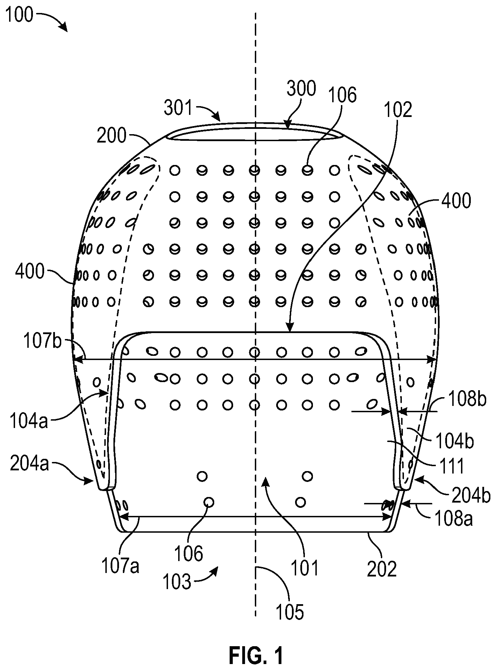

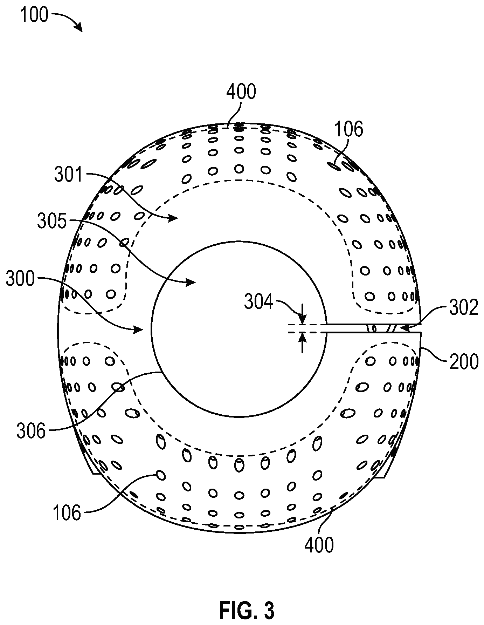

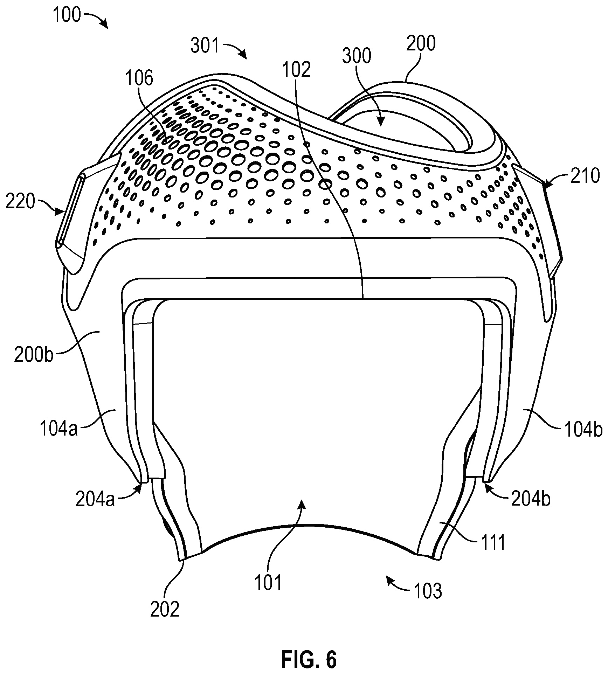

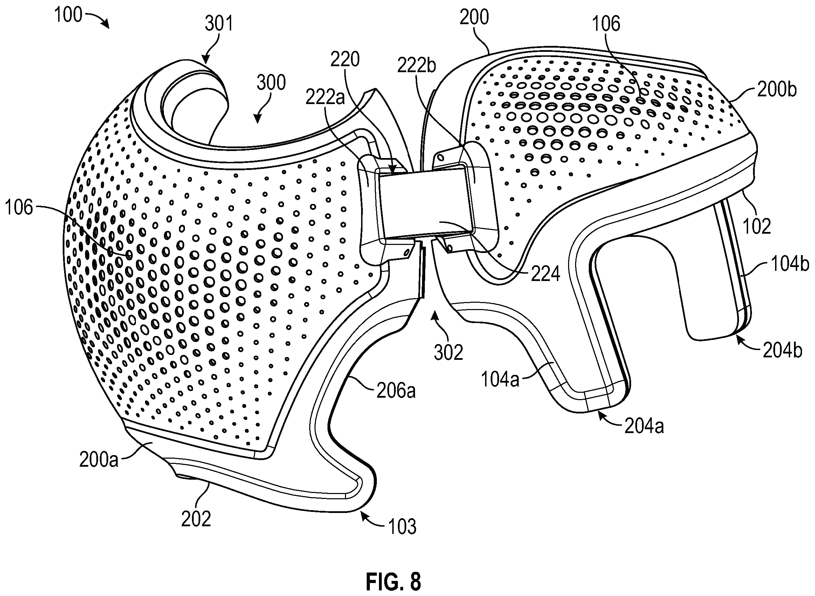

Referring particularly to FIGS. 1-9, a helmet 100 (e.g., an orthotic, a cranial orthotic, a cranial remodeling orthotic, an orthotic device, a pediatric orthotic device, a cranial orthotic device, a cranial orthotic remodeling device, a cranial remodeling device, a cranial helmet, a helmet, etc.) is configured for use with a head of a pediatric patient or user, according to some embodiments. Helmet 100 can be configured for use with patients whose skulls are asymmetrically distorted because of conditions such as brachycephaly, plagiocephaly, scaphocephaly, etc. Helmet 100 can be placed onto the patient's head to provide proper distribution of forces across the head and to provide stability for the head when helmet 100 is worn and used. Helmet 100 can include areas of void when in use (e.g., when helmet 100 is worn by the patient) configured to allow growth in one or more directions to result in a more symmetric head shape over time, and can include areas of contact when in use to limit growth in one or more directions to result in a more symmetric head shape over time. A duration of treatment (e.g., helmet therapy) depends on individual needs of the patient, but can include the patient wearing the helmet 100 for one month to six months. Helmet 100 can be manufactured, fabricated, or constructed using additive manufacturing techniques such as 3d printing.

Referring still to FIGS. 1-9, helmet 100 can include a shell, an outer shell, a structural member, an exterior wall, an exterior shell, etc., shown as shell 200. Shell 200 can include an inner volume 101 (e.g., a void, a cavity, etc.) configured to receive the patient's head. The patient may insert their head into the inner volume 101 of shell 200 at a lower or distal end 103 of shell 200. In some embodiments, contours of shell 200 are configured to align with anatomical contours of the patient's head. Shell 200 can be configured to surround, enclose, or fully receive the patient's head. Shell 200 can include a hole or opening 300 at an upper or proximal end 301 of shell 200 configured to be positioned on the top of the patient's head. Opening 300 can correspond to any rounded shape (e.g., circular, elliptical, etc.) shown as circle 305 with circumference 306. In some embodiments, a geometry of shell 200 (e.g., a shape of inner volume 101) corresponds to or matches a desired shape or geometry of the patient's head.

Shell 200 can include a first temporal extension 104a and a second temporal extension 104b configured to fit the anatomical structure of the patient's head and extend down towards a patient's cheeks or jawline on both sides of a patient's face. Shell 200 can also include trimlines such as anterior trimline 102 along the front of shell 200, occipital trimline 202 along the distal end 103 of shell 200, first aural trimline 206a along one side of shell 200 and second aural trimline 206b along an opposite side of shell 200 from first aural trimline 206a. Anterior trimline 102 can be configured to lay across a patient's forehead near a patient's brow line. Occipital trimline 202 can be configured to lay near a nape of a patient's neck. First aural trimline 206a and second aural trimline 206b can be configured to create an opening or a gap in shell 200 for a patient's ears. In some embodiments, the gap extends down towards the occipital trimline 202 on a rear side of the gap and towards either a first inferior aspect 204a of the first temporal extension 104a or a second inferior aspect 204b of the second temporal extension 104b on the opposite side of the gap.

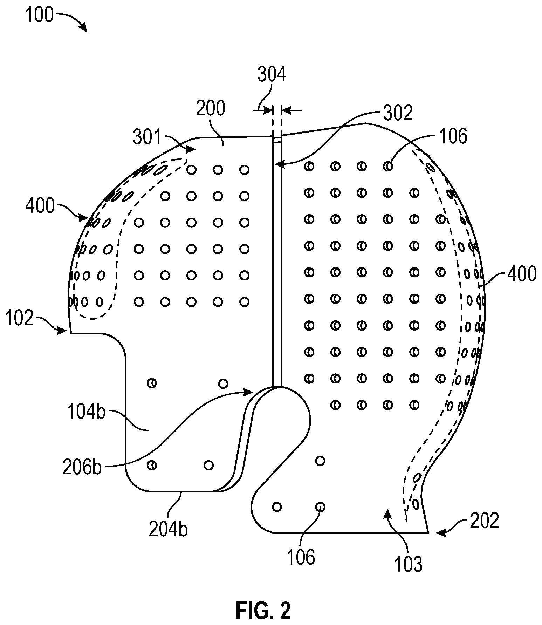

Referring particularly to FIGS. 1-3, shell 200 can include a side opening 302 along one side of helmet 100. Side opening 302 can extend from opening 300 at the proximal end 301 of helmet 100 down towards either the first aural trimline 206a or the second aural trimline 206b (e.g., the aural trimline on the same side of helmet 100 as the side opening). For example, FIG. 2 shows side opening 302 extending from opening 300 at the proximal end 301 of helmet 100 down towards the second aural trimline 206b. In some embodiments, side opening 302 can have a width 304 depending on the anatomical structure and needs of the patient.

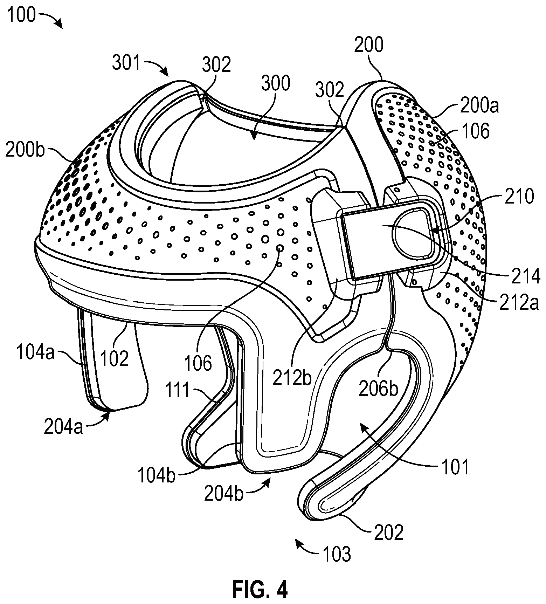

As shown in FIGS. 4-11, head orthotic device or helmet 100 can include a first half 200a and a second half 200b of shell 200. First half 200a and second half 200b refer to two pieces of shell 200 that, when coupled together as described herein, form shell 200 in some embodiments. In some examples, shell 200 may include side openings 302 between the first half 200a and the second half 200b along both sides of helmet 100. A first side opening 302 between the first half 200a and the second half 200b can extend from opening 300 at the proximal end 301 of helmet 100 down towards the first aural trimline 206a, and a second side opening 302 between the first half 200a and the second half 200b can extend from opening 300 at the proximal end 301 of helmet 100 down towards the second aural trimline 206b in some embodiments.

In some embodiments, the shell 200 includes a fastener configured to close side opening 302. In this way, the fastener may be used to secure helmet 100 on a patient's head. In some embodiments, where shell 200 has side opening 302 along one side of helmet 100, as shown in FIGS. 1-3, shell 200 includes a fastener on the same side of helmet 100 as side opening 302. Where shell 200 has side openings 302 along both sides of helmet 100, the shell 200 can include a fastener on both sides of helmet 100 such that the fasteners are configured to close side openings 302 along both sides of helmet 100.

In some embodiments, where shell 200 has side openings 302 along both sides of helmet 100, shell 200 can include clasp mechanism 210 configured to close a first side opening 302 between the first half 200a and the second half 200b and hinge mechanism 220 configured to close a second side opening 302 between the first half 200a and the second half 200b. For example, as shown in FIGS. 4-9, clasp mechanism 210 can be used to close side opening 302 extending from opening 300 at the proximal end 301 of helmet 100 down towards the second aural trimline 206b, and hinge mechanism 220 can be used to close side opening 302 extending from opening 300 at the proximal end 301 of helmet 100 down towards the first aural trimline 206a.

According to various embodiments, shell 200 can be manufactured as a single piece with integrated features. For example, shell 200 can include integrated closure mechanisms (e.g., 3D printed zippers, 3D printed knob and post attachments, integrated lace eyelets, 3D printed snaps, 3D printed dovetailing or mechanical interlocks, 3D printed living hinges, etc.). In this way, shell 200 can be manufactured to include the clasp mechanism 210 and/or the hinge mechanism 220 as an integrated feature. In some embodiments, the clasp mechanism 210 and/or the hinge mechanism 220 may be additively manufactured as components of shell 200, as described herein. Furthermore, the use of different materials also allows for the printing of springs (e.g., springs 230, as described below) with 3D printed material (e.g., plastic coil or leaf springs for compression or expansion applications between items that need to be separated with a spring connection, articulations or "living hinges" within the device without separating the device into multiple components or adding additional external hardware, and the like). Size, materials, and configurations of mechanisms 210 and 220 can be chosen according to the scan discussed with reference to process 500. A library of constructions for mechanisms 210 and 220 can be stored. Configurations such as left handed or right handed operative configurations can be selected in some embodiments.

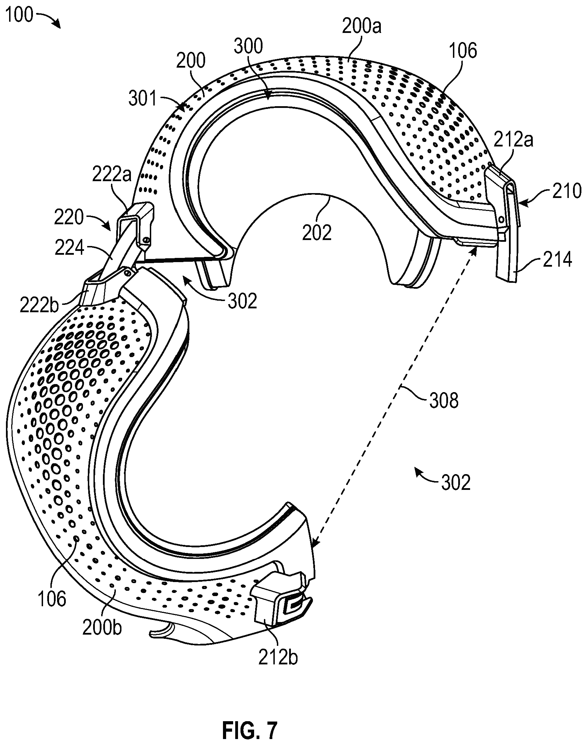

Referring to FIGS. 4-9, the clasp mechanism 210 and the hinge mechanism 220 are configured to convert helmet 100 between a closed configuration (e.g., as shown in FIGS. 4-6) and an open configuration (e.g., as shown in FIGS. 7-9). For example, helmet 100 may be in the open configuration while being put on a patient's head. Then, once helmet 100 is placed on the patient's head, clasp mechanism 210 and hinge mechanism 220 can be used to convert helmet 100 to the closed configuration, therefore securing helmet 100 secured on the patient's head for therapy. Similarly, clasp mechanism 210 and hinge mechanism 220 can be used to convert helmet 100 from the closed configuration to the open configuration, such as when helmet 100 is being removed from the patient's head. As shown in FIGS. 7-9, clasp mechanism 210 and hinge mechanism 220 may be configured to expand side opening 302 on the same side of helmet 100 as clasp mechanism 210 by a distance 308. In this way, when helmet 100 is in the open configuration, one of the side openings 302 becomes wide enough to receive a patient's head, thereby facilitating placement of helmet 100 on the patient's head or removal of helmet 100 from the patient's head without asserting unintentional pressure on the patient's head from helmet 100.

In some embodiments, first half 200a of shell 200 includes a first socket 212a configured to receive a first end of clasp mechanism 210, and second half 200b of shell 200 includes a second socket 212b configured to receive a second end of clasp mechanism 210. More specifically, the first socket 212a may receive a fixed end of clasp mechanism 210, while the second socket 212b may receive a removable end of clasp mechanism 210. In other words, in the open configuration, the fixed end of clasp mechanism 210 can remain coupled to the first half 200a, while the removable end of clasp mechanism 210 is released from the second half 200b. Similarly, first half 200a of shell 200 can include a first socket 222a configured to receive a first end of hinge mechanism 220, and second half 200b of shell 200 can include a second socket 222b configured to receive a second end of hinge mechanism 220. As described in greater detail below, the first end of hinge mechanism 220 remains coupled to the first half 200a and the second end of hinge mechanism 220 remains coupled to the second half 200b whether helmet 100 is in the open configuration (e.g., FIGS. 7-9) or in the closed configuration (e.g., FIGS. 4-6).

According to some embodiments, clasp mechanism 210 can include interfacing member 214. During transition of helmet 100 from the closed configuration to the open configuration, for instance, a user (e.g., a medical professional, a parent of the patient, etc.) can engage with the interfacing member 214, as described below, to detach the interfacing member 214 from the second half 200b of shell 200 at the removable end of clasp mechanism 210, while interfacing member 214 remains coupled to the first half 200a of shell 200 at the fixed end of clasp mechanism 210. In some embodiments, interfacing member 214 includes a recess where the user can engage with the interfacing member 214. This recess of the interfacing member 214 provides an ergonomic benefit to users engaging with the interfacing member 214 of clasp mechanism 210.

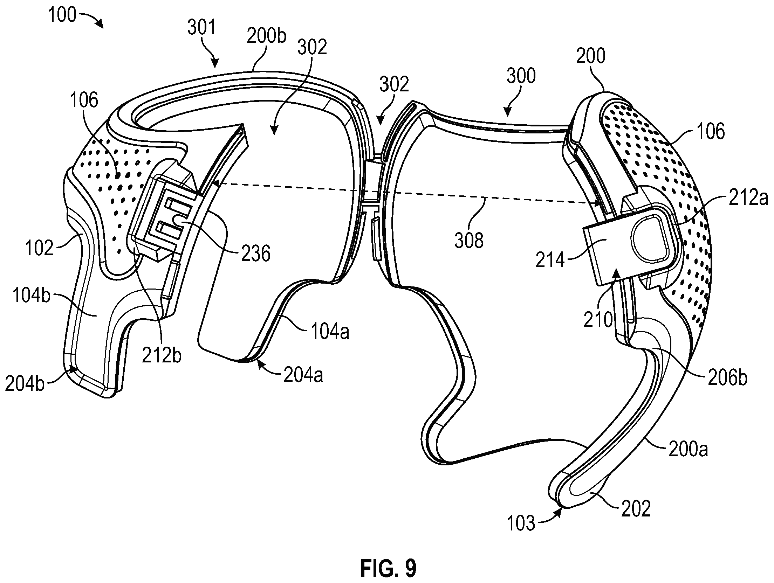

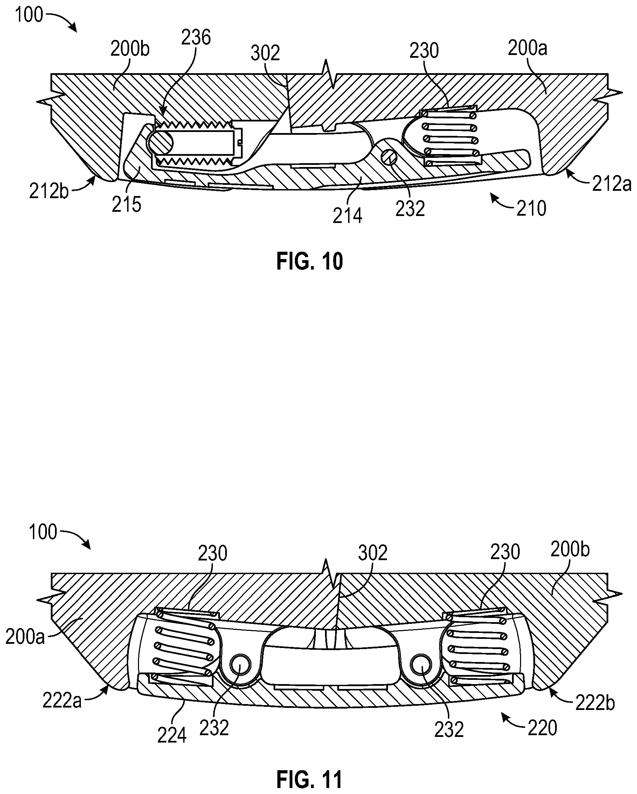

Referring to FIGS. 9 and 10, clasp mechanism 210 can include a hooked end 215 of interfacing member 214 configured to engage with a detent mechanism 236. In some embodiments, hooked end 215 can be received by second socket 212b of second half 200b. Detent mechanism 236 can refer to a spring-loaded detent mechanism configured to release hooked end 215 of interfacing member 214 from second socket 212b during the transition between the open and closed configuration of helmet 100. For instance, the user may engage with (e.g., apply a force upon, press down on, push, etc.) the interfacing member 214 at the fixed end of the clasp mechanism 210 (e.g., the end that is opposite from hooked end 215). As shown in FIG. 10, clasp mechanism 210 can include a spring 230 coupled to shell 200. Therefore, when the user engages with the interfacing member 214, the spring 230 is compressed and the interfacing member 214 is configured to rotate about pivot point 232. Rotation of interfacing member 214 about pivot point 232 can cause hooked end 215 of interfacing member 214 to engage with detent mechanism 236, and the detent mechanism 236 is configured to release the hooked end 215 of interfacing member 214 from the second socket 212b. Once the hooked end 215 of the interfacing member 214 is released, the interfacing member 214 is detached from the second half 200b of shell 200, thus widening the side opening 302 between the first half 200a and the second half 200b of shell 200 on the side of helmet 100 with clasp mechanism 210.

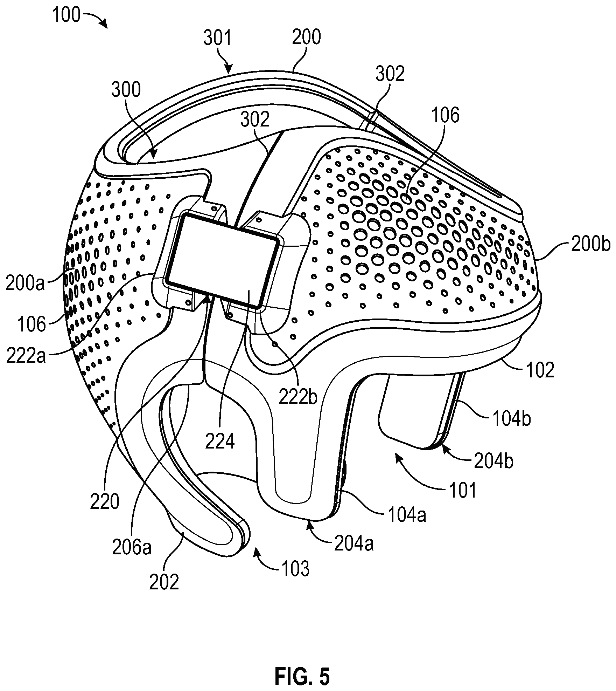

Referring to FIGS. 5-11, hinge mechanism 220 includes a frontal member 224, two springs 230, and two pivot points 232. Frontal member 224 is coupled to first half 200a of shell 200 at first socket 222a and is coupled to second half 200b at second socket 222b. In some embodiments, when the side opening 302 between the first half 200a and the second half 200b is widened on the side of helmet 100 with the clasp mechanism 210, hinge mechanism 220 can be used to widen the side opening 302 on the side of helmet 100 with the hinge mechanism 220 such that the first half 200a and the second half 200b of shell 200 do not collide with each other. That is, when the hooked end 215 of interfacing member 214 is released, first half 200a and second half 200b of shell 200 rotate towards each other on the side of helmet 100 opposite from the clasp mechanism 210 (e.g., as the side of helmet 100 with hinge mechanism 220). Therefore, as shown in FIG. 11, the first half 200a and the second half 200b of shell 200 can engage with frontal member 224 at both ends of frontal member 224. In some embodiments, a force applied to both ends of frontal member 224 causes frontal member 224 to engage with springs 230. In some embodiments, springs 230 can be biasing at closed such that when the frontal member 224 engages with the springs 230, the springs 230 are configured to release outward from shell 200. When the springs 230 are released, hinge mechanism 220 can rotate about the pivot points 232 such that the side opening 302 on the side of helmet 100 with the hinge mechanism 220 widens.

Referring now to FIGS. 1-12B, helmet 100 can include pads, interior pads, internal padding, inner pads, internal foam pads, etc., shown as pads 400. In some embodiments, shell 200 includes an array of a plurality of peg holes, receivers, openings, etc., shown as peg holes 106. In some embodiments, the array of the plurality of peg holes 106 is a two-dimensional array. Peg holes 106 can be configured to receive pads 400 from an inwards facing surface or interior surface 111 of shell 200. In some embodiments, a shape and/or a size of peg holes 106 can be uniform across the shell 200. In some embodiments, the shape and/or the size of peg holes 106 can be non-uniform or varying across the shell 200. In some embodiments, peg holes 106 can be arranged in any pattern configured to receive the pads 400 required to achieve the desired head shape of the patient. In some embodiments, receiving the pads 400 includes press-fitting the pads 400 into peg holes 106 of shell 200.

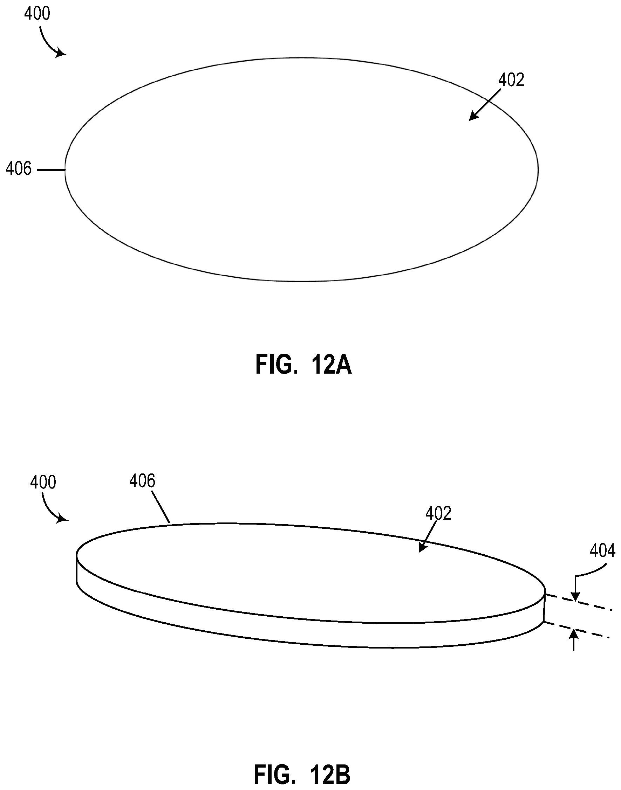

Referring particularly to FIGS. 12A-12B, pads 400 can assume any shape (e.g., circular, elliptical, rectangular, triangular, etc.) shown as shape 402. Shape 402 can depend on the needs of the patient, a size of the patient's head (e.g., depending on an age of the patient), the desired head shape, etc. In some embodiments, pads 400 are designed from a library of shapes. Shape 402 has a perimeter 406 (e.g., a circumference, an outer periphery, etc.) depending on the size of pads 400 needed in helmet 100 to achieve the desired head shape, according to some embodiments. Shape 402 and/or perimeter 406 can be consistent or uniform among the pads 400 used in helmet 100 or can vary between the pads 400 used in helmet 100. Helmet 100 can utilize any number of pads 400 required to achieve the desired head shape and to increase patient comfort. Pads 400 can either be additively manufactured (e.g., 3d printed), injection molded, or identified as standard or pre-manufactured parts.

Referring still to FIGS. 12A-12B, pads 400 can have thickness 404 designed to create the areas of void or the areas of contact within the helmet 100 depending on the anatomical structure of the patient and the desired head shape. Thickness 404 can be consistent or uniform across the number of pads 400 used in helmet 100 or can vary across the number of pads 400 used in helmet 100. Thickness 404 can also be consistent or uniform across any one of pads 400 or can vary across any one of pads 400. For example, thickness 404 of pads 400 may be greater in areas of helmet 100 that require an area of contact in order to limit skull growth in a particular direction. In some embodiments, thickness 404 of pads 400 may be less in areas of helmet 100 that require an area of void to allow skull growth in a particular direction. Thickness 404 of pads 400 can be greater in areas of helmet 100 that are expected to experience a greater amount of stress (e.g., areas of the helmet 100 that may experience the most contact with an external surface when the patient sleeps) so as to maintain patient comfortability while the helmet 100 is in use. In some embodiments, additive manufacturing can be used to produce components of helmet 100 with variable thickness.

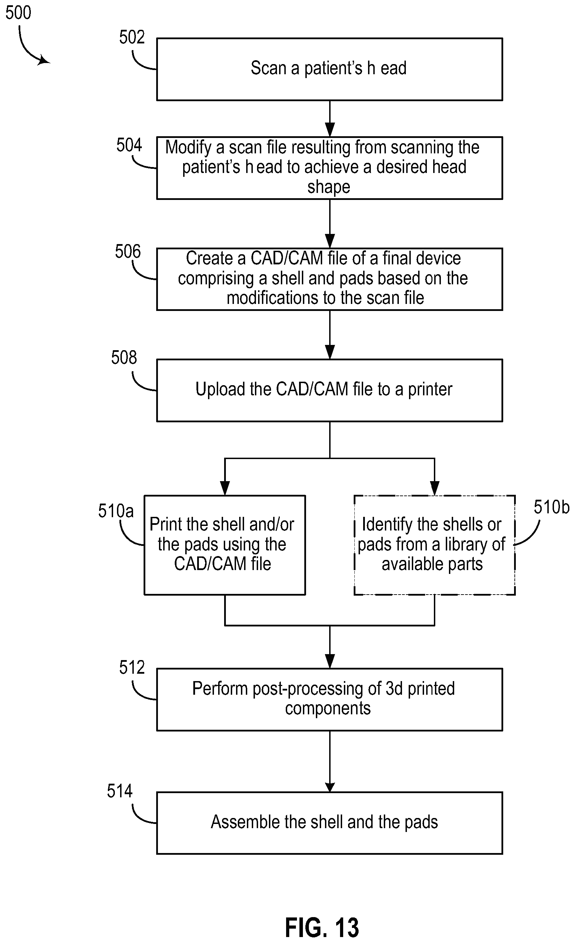

In some embodiments, pads 400 are manufactured or produced from a material such as foam (e.g., open cell polyurethane, closed cell polyethylene, rubber, etc.). In some embodiments, the foam can be custom fit to an anatomical structure of the patient's head. A material composition of the material of pads 400 can be lightweight for improved patient comfort. In some embodiments, the material composition of pads 400 facilitates minor adjustments to be made to an overall shape of pads 400 by heating the pads 400. Pads 400 can be heated in particular areas where a plastic deformation is desired, deformed (e.g., by a manufacturer) and cooled so that the deformation remains. In this way, pads 400 can be adjusted or deformed plastically (or elastically, without heat addition) without sustaining structural damage. For example, pads 400 can be modified (e.g., by adding heat and applying a force) to account for any potential growth in areas of the patient's head over the course of treatment. In some embodiments, pads 400 can be replaced after taking an updated scan of the patient's head (step 502 of process 500 described in greater detail below with reference to FIG. 13). New pads 400 can have updated thicknesses, shape, size, etc., to account for progression in a patient's treatment towards increased symmetry of the patient's head shape. In some embodiments, an original fabrication of pads 400 can be updated (e.g., remove excess material, alter shape, reduce thickness, etc.) to accommodate for growth and progression towards the desired head shape.

Referring now to FIGS. 1-9, shell 200 is manufactured or produced from a material such as a thermoplastic (e.g., a versatile thermoplastic such as nylon). A material composition of the material of shell 200 can be lightweight for improved patient comfort. In some embodiments, the material composition of shell 200 facilitates variable flexibility and rigidity throughout shell 200 (e.g., along a height of shell 200 from the proximal end 301 to the distal end 103). A longitudinal axis, a central axis, a centerline, or a dimension can be defined between the proximal end 301 and the distal end 103. For purposes of illustration, FIG. 1 includes a centerline 105 extending through shell 200.

In some embodiments, the material composition of shell 200 facilitates minor adjustments to be made to an overall shape of shell 200 by heating the shell 200. Shell 200 can be heated in particular areas where a plastic deformation is desired, deformed (e.g., by a manufacturer) and cooled so that the deformation remains. In this way, shell 200 can be adjusted or deformed plastically (or elastically, without heat addition) without sustaining structural damage. For example, shell 200 can be modified (e.g., by adding heat and applying a force) to account for any growth in areas of the patient's head over the course of treatment.

In some embodiments, and as shown in FIG. 1, shell 200 may include one or more dimensions such as a first width 107a of inner volume 101, a first thickness 108a of shell 200, a second width 107b of inner volume 101, a second thickness 108b of shell 200, various circumferences, etc. It should be understood that first thickness 108a and second thickness 108b both show the thickness of shell 200 but at different orientations and different positions along the height of helmet 100. In some embodiments, the thickness (e.g., first thickness 108a and/or second thickness 108b) of shell 200 is constant or uniform along the height of helmet 100. In some embodiments, the thickness of shell 200 is non-constant along the height of helmet 100 and is instead variable. For example, the thickness of shell 200 may be greatest at the proximal end 301 of helmet 100 and decrease to a lowest value at the distal end 103 of helmet 100. It should be understood that any number of thicknesses of shell 200 can be defined taken from any orientation of shell 200 (e.g., at any view, at a view 45 degrees between the front view and the side view, etc.). By providing heat and applying forces or moments to shell 200, one or more of the dimensions can be adjusted. For example, a curvature of shell 200 at a base of shell 200 of helmet 100 (e.g., at distal end 103) can be adjusted by applying heat and plastically deforming the shell 200.

It should also be understood that the thickness of shell 200 may vary at different orientations or angles relative to centerline 105 or a longitudinal axis extending through helmet 100. In this way, different areas or portions of shell 200 (e.g., different locations along the height of helmet 100, or along centerline 105, or along the longitudinal axis, etc.) can have different thicknesses. The different thicknesses can correspond to an amount of deformation (e.g., plastic or elastic) or flexion (e.g., plastic or elastic) that the shell 200 experiences (during use of the helmet 100 or when heat is applied to adjust the geometry of helmet 100). In some embodiments, areas where shell 200 is thinner (e.g., the thickness is at a decreased value) experience greater degrees or amounts of deformation or flexion. Similarly, areas where shell 200 is thicker (e.g., the thickness is at an increased value) experience a smaller degree or amount of deformation or flexion relative to the thinner areas, according to some embodiments. In some embodiments, the thickness of shell 200 (e.g., first thickness 108a and/or second thickness 108b) is designed or configured to provide desired flexion or deformation when used by the patient to improve symmetry of the patient's head and/or comfortability of the helmet 100.

Referring still to FIGS. 1-9, shell 200 may taper (e.g., decreasing thickness) at anterior trimline 102, occipital trimline 202, first aural trimline 206a or second aural trimline 206b. In some embodiments, anterior trimline 102, occipital trimline 202, first aural trimline 206a or second aural trimline 206b can be adjusted (e.g., by applying heat and plastically deforming shell 200, or during the manufacturing/design process of shell 200) to fit the requirements of the patient's skull and to increase comfortability of helmet 100 when in use.

In some embodiments, helmet 100 is configured to surround and contain the patient's skull. Helmet 100 thereby provides stability across the patient's skull and provides a proper distribution of forces when in use with the skull. Helmet 100, along with the specific geometry that is patient-specific (e.g., shell 200 and pads 400) can be achieved through a fabrication or manufacturing process such as additive manufacturing, described in greater detail below.

### Additive Manufacturing Process

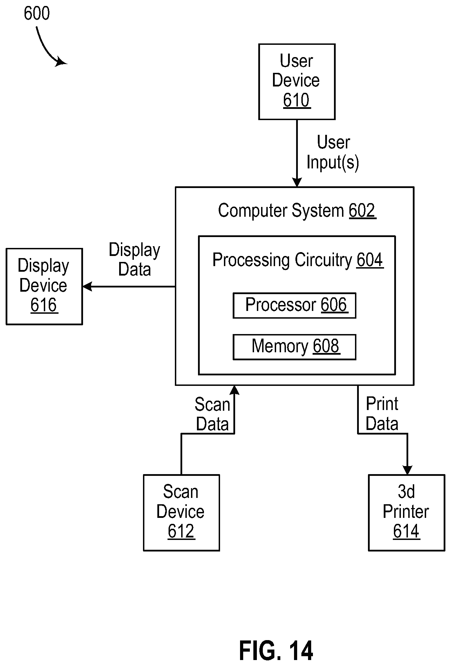

Referring particularly to FIG. 13, a flow diagram of a process 500 for producing or manufacturing the helmet 100 of FIGS. 1-12B is shown, according to some embodiments. Process 500 includes steps 502-514 and can be performed using an additive manufacturing system (e.g., system 600 as described in greater detail below with reference to FIG. 14).

Process 500 includes scanning a patient's head (step 502), according to some embodiments. In some embodiments, step 502 is performed using a scan device, 3d scanner, digital scanner, etc. (e.g., scan device 612 as described in greater detail below with reference to FIG. 14). In some embodiments, performing step 502 results in the generation of a scan file. The scan file can capture an anatomical structure of the patient's head. In some embodiments, the anatomical structure can be used to create a custom design of helmet 100 fit for the patient.

Process 500 includes modifying a patient scan file resulting from the scan (e.g., resulting from performing step 502) to a 3d model of a custom device (e.g., the helmet 100) fit to a desired head shape for the patient (step 504), according to some embodiments. The 3d model of the device can include components of the helmet 100 such as the shell 200 and pads 400 configured to achieve the desired head shape when worn over time. In some embodiments, step 504 is performed on a computer system based on one or more user inputs or inputs from a health care provider (e.g., computer system 602 as described in greater detail below with reference to FIG. 14). For example, step 504 can include adjusting a thickness (e.g., first thickness 108a of shell 200, second thickness 108b of shell 200, thickness 404 of pads 400, etc.) of the device of the scan file at different locations. The modifications performed in step 504 can include but are not limited to build-ups, reductions, smoothing, adjustments, etc.

In some embodiments, step 504 includes digitally using buildups or reductions to the thickness of the 3d model of the device to achieve a desired thickness. For example, the thickness may be configured to create an area of void when the device is worn by the patient to allow growth in a particular direction in order to achieve the desired head shape. In some embodiments, the thickness may be configured to create an area of contact with the patient's head when the device is worn by the patient to limit growth in a particular direction in order to achieve the desired head shape. In some embodiments, step 504 can be performed by computer system 602 based on one or more user inputs or inputs from a health care provider obtained from user device 610 (described in greater detail below with reference to FIG. 14).

Process 500 includes creating a design file (e.g., a computer assisted design (CAD) file and/or a computer assisted manufacturing (CAM) file) of the device including shell 200 and pads 400 based on the modifications to the scan file (step 506), according to some embodiments. Process 500 also includes uploading the CAD or CAM file to a printer (e.g., 3d printer 614) (step 508), according to some embodiments. Step 506 and step 508 can be performed by computer system 602 (e.g., in response to a user input such as from a health care provider).

Process 500 includes printing at least one of the components in the CAD or CAM file using 3d printing (e.g., shell 200 and/or pads 400) (step 510a), according to some embodiments. In some embodiments, step 510a includes performing additive manufacturing (e.g., dispensing or outputting layers consecutively on top of each other) to produce at least one of the components of the device. In some embodiments, the additive manufacturing of the shell 200 is performed using a single uniform material such as a thermoplastic (e.g., nylon). In some embodiments, the additive manufacturing of the pads 400 is performed using a single uniform material such as foam. The resulting 3d printed component or components (e.g., shell 200 and/or pads 400) can have variable thickness as defined by the CAD or CAM file.

In some embodiments, process 500 includes identifying at least one of the components of the CAD or CAM file from a library of available parts (e.g., shell 200 and/or pads 400) (step 510b). If either the shell 200 and/or pads 400 of the CAD or CAM file are available as standard or pre-manufactured components, process 500 will identify a product that corresponds with the component from the CAD or CAM file (step 510b). If neither the shell 200 nor the pads 400 of the CAD or CAM file are available as a standard or pre-manufactured component, the entire device (e.g., shell 200 and pads 400) can be produced by additive manufacturing (step 510a). In some embodiments, if the pads 400 of the CAD or CAM file are not available as a standard of pre-manufactured component, the pads 400 can be injection molded or additively manufactured to produce the device.

Process 500 includes performing post-processing of the 3d printed components (step 512), according to some embodiments. For example, step 512 can include removing excess material that is dispensed during step 510a (e.g., during fabrication of at least one of the components of the device). Step 512 can be performed by a technician. Additional post-processing can be performed based on anatomy or needs of the patient. For example, step 512 can include adjusting trimlines (e.g., anterior trimline 102, occipital trimline 202, first aural trimline 206a, second aural trimline 206b, etc.), opening 300, or side opening 302 of shell 200 to produce the component of the device in the modified scan file of step 504.

Process 500 includes assembling the components (e.g., the shell 200 and pads 400) into the final device (e.g., helmet 100). Pads 400 are configured to be received by the peg holes 106 of shell 200. Pads 400 can be produced through an additive manufacturing process (e.g., by 3d printer 614) (step 510a), can be manufactured by an injection molding process, or can be identified as a standard or pre-manufactured part or component from a library of available parts (step 510b). Similarly, shell 200 can be additively manufactured (e.g., by 3d printer 614) (step 510a) or can be identified as a standard of pre-manufactured part or component from a library of available parts (step 510b).

In some embodiments, the device that is produced by performing the process 500 is a custom pediatric orthotic device, with a varying thickness (e.g., cross-sectional thickness) throughout. In some embodiments, the device includes individual components such as an outer shell and internal padding. The device can provide proper stability and distribution of forces when worn, and can be produced using additive manufacturing techniques. In some embodiments, the device is constructed using a combination of additively manufactured and standard of pre-manufactured parts or components identified from a library of available parts. The thickness of the device can be modified in any area to accommodate the anatomy of the patient as well as any additional requirements the patient may have (e.g., based on input from a health care provider). The device is created using 3d printing, where the material composition of each component (e.g., outer shell and internal padding) can be of a single uniform substance and can provide extra comfort to the patient when worn due to its lightweight properties, according to some embodiments.

### Additive Manufacturing System Architecture

Referring now to FIG. 14, a system 600 for additive manufacturing of prosthetic, orthotic, or protective devices is shown, according to some embodiments. System 600 includes a user device 610, a display device 616, a computer system 602, a scan device 612, and an additive manufacturing device or 3d printer 614.

Computer system 602 is configured to receive scan data from scan device 612, according to some embodiments. Computer system 602 can be a desktop computer, a laptop, a remote computing system, etc. Computer system 602 includes processing circuitry 604 having memory 608 and a processor 606. Processor 606 can be implemented as a general-purpose processor, an application specific integrated circuit (ASIC), one or more field programmable gate arrays (FPGAs), a group of processing components, or other suitable electronic processing components.

Memory 608 (e.g., memory, memory unit, storage device, etc.) may include one or more devices (e.g., RAM, ROM, Flash memory, hard disk storage, etc.) for storing data and/or computer code for completing or facilitating the various processes, layers and modules described in the present application. Memory 608 may be or include volatile memory or non-volatile memory. Memory 608 may include database components, object code components, script components, or any other type of information structure for supporting the various activities and information structures described in the present application. According to an exemplary embodiment, memory 608 is communicably connected to processor 606 via processing circuitry 604 and includes computer code for executing (e.g., by processing circuitry 604 and/or processor 606) one or more processes described herein.

Computer system 602 can be configured to run CAD computer software to facilitate the design and production of the device. Computer system 602 is configured to receive scan data from scan device 612, according to some embodiments. In some embodiments, the scan data is a scan file obtained from scan device 612. In some embodiments, a technician may use scan device 612 to scan a patient's head, thereby generating the scan data.

When the scan data is provided to computer system 602, computer system 602 can generate a CAD or CAM file. In some embodiments, the CAD or CAM file includes a visual representation of a 3d model of the components (e.g., outer shell and inner padding) of the orthotic device. A user (e.g., a health care provider) can then provide inputs (e.g., via user device 610) to adjust geometry, thickness, etc., of the 3d model in the CAD or CAM file. More generally, computer system 602 may use the scan data to generate a digital representation of a device to be manufactured for the patient's head. Computer system 602 can provide display data to display device 616 (e.g., a computer screen, a display screen, etc.) so that the digital representation is visually displayed in real-time. The user or health care provider can then view real-time changes or updates as the user changes or adjusts the CAD or CAM file.

For example, the user may adjust the CAD or CAM file so that the design gradually tapers or thickens in different areas. In some embodiments, the user may decrease thickness of one or both of the components (e.g., outer shell and/or inner padding) of the CAD or CAM file to create an area of void when the device is worn by the patient to allow skull growth in a particular direction. The user may also increase thickness to create an area of contact with the patient's head when the device is worn by the patient to limit skull growth in a particular direction. In some embodiments, the user or the health care provider may use data from different experiments to identify areas where a patient may experience high stress. The user may increase thickness of one or both of the components of the CAD or CAM file at areas where high stress is experienced so that the 3d printed device may maintain its intended shape and geometry. In some embodiments, thickness of the 3d printed device is maintained above a minimum thickness value. The user can also use knowledge regarding different cranial contours of the patient to determine which areas of the 3d model in the CAD or CAM file should have decreased or increased thickness in order to promote patient comfort. The user may also use historical data to determine which areas or portions of the 3d model or the CAD or CAM file should have increased or decreased thickness (e.g., wall thickness).

Once the user (e.g., the health care provider) has adjusted or manipulated the CAD or CAM file, the user can prompt the computer system 602 to export the file to 3d printer 614 as print data. Computer system 602 can convert the adjusted, manipulated, or updated CAD or CAM file to a file type that is compatible with 3d printer 614 (e.g., a Standard Tessellation Language (STL) file). Computer system 602 then provides the print data to 3d printer 614.

The 3d printer 614 can be any additive manufacturing machine or device that is configured to successively provide or discharge layers of material onto each other to form or construct a part or components. 3d printer 614 may be configured to dispense material (e.g., one or more powder materials that can form nylon when combined with fusing/detailing agents and exposed to fusing light, or any other dispensable materials) in layers to fabricate at least one component (e.g., outer shell and/or inner padding) of the CAD or CAM file.

Advantageously, the systems and methods described herein can be used to produce 3d printed prosthetics, orthotics, or protective devices. Traditional molding methods do not offer the same precision for pad attachment as does additive manufacturing. The precise locations of the pad attachment can be achieved using additive manufacturing by producing a shell or head worn structure having the holes or openings at locations for receiving the pads. The systems and methods described herein that use additive manufacturing can facilitate improved fit, comfort, and cranial remodeling by providing more precise positioning and sizing of pads.

### Recommendation System

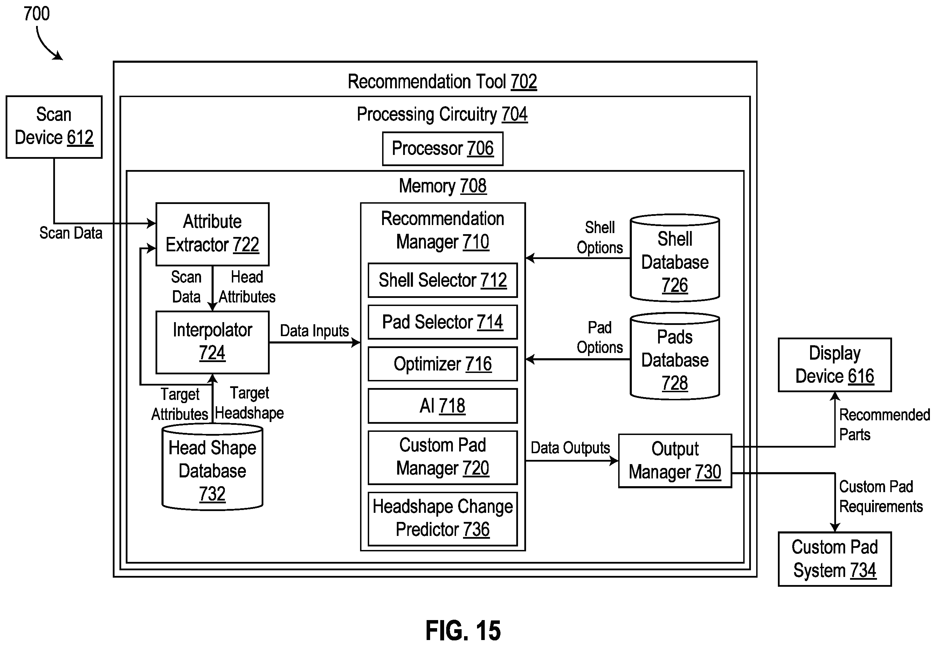

Referring to FIG. 15, a recommendation system 700 includes a recommendation tool 702, the scan device 612, the display device 616, and a custom pad system 734, according to some embodiments. In some embodiments, the recommendation tool 702 is configured to receive the scan data from the scan device 612, and select a recommended model of the shell 200 and recommended pads 400. The recommendation system 700 can use the scan data from the scan device 612 to determine (e.g., by performing an algorithm, an optimization, or a deep learning technique) the recommended model of the shell 200 and the recommended pads 400. In some embodiments, the recommendation tool 702 is configured to provide a recommendation including placement of the recommended pads 400 at specific locations within the recommended model of the shell 200. The shell 200, or the variety of models of the shell 200, and the pads 400 can be provided as a kit. In some embodiments, the recommendation system 700 performs a decision making process to select, based on the scan data provided by the scan device 612, a recommended shell 200 from the kit, as well as recommended pads 400, and locations for placement of the recommended pads 400 within the recommended shell 200.

The recommendation system 700 can be implemented on a desktop computer, a laptop, a remote computing system, etc. The recommendation tool 702 includes processing circuitry 704 having memory 708 and a processor 706. Processor 706 can be implemented as a general-purpose processor, an application specific integrated circuit (ASIC), one or more field programmable gate arrays (FPGAs), a group of processing components, or other suitable electronic processing components.

Memory 708 (e.g., memory, memory unit, storage device, etc.) may include one or more devices (e.g., RAM, ROM, Flash memory, hard disk storage, etc.) for storing data and/or computer code for completing or facilitating the various processes, layers and modules described in the present application. Memory 708 may be or include volatile memory or non-volatile memory. Memory 708 may include database components, object code components, script components, or any other type of information structure for supporting the various activities and information structures described in the present application. According to an exemplary embodiment, memory 708 is communicably connected to processor 706 via processing circuitry 704 and includes computer code for executing (e.g., by processing circuitry 704 and/or processor 706) one or more processes described herein.

The memory 708 includes an attribute extractor 722, an interpolator 724, a head shape database 732, a recommendation manager 710, a shell database 726, a pads database 728, and an output manager 730, according to some embodiments. It should be understood that the attribute extractor 722, the interpolator 724, the head shape database 732, the recommendation manager 710, the shell database 726, the pads database 728, or the output manager 730 as described herein may represent stored instructions that are implemented by the processor 706 and/or the processing circuitry 704. It should further be understood that any of the techniques, processes, algorithms, calculations, determinations, etc., described herein with reference to the recommendation tool 702 may be implemented locally as a program, or at least partially remotely (e.g., via a computer system that communicates with a cloud based computing system or server).

The attribute extractor 722 is configured to obtain the scan data from the scan device 612 and identify one or more attributes of the patient's head shape, according to some embodiments. For example, the attribute extractor 722 may identify an overall size of the patient's head shape such as diameter, circumference, radius, volume, etc. In some embodiments, the attribute extractor 722 is also configured to identify one or more deviations of the patient's head relative to a target head shape. The attribute extractor 722 may receive one or more target attributes or target head shapes from the head shape database 732 and identify deviations of the patient's head shape as indicated by the scan data. The deviations of the patient's head shape relative to a target head shape may indicate a condition of the patient such as brachycephaly, plagiocephaly, scaphocephaly, etc. In some embodiments, the head shape database 732 includes scan data of patients that have been diagnosed with brachycephaly, plagiocephaly, scaphocephaly, etc. The attribute extractor 722 may perform a matching technique in order to identify if the patient's head resembles the scan data of patients that have been diagnosed with brachycephaly, plagiocephaly, scaphocephaly, etc.

In some embodiments, the attribute extractor 722 is configured to use a grid including multiple quadrants to identify locations or zones of the patient's head (based on the scan data) that deviate from the target head shape. In some embodiments, the attribute extractor 722 is configured to use a defined coordinate system that corresponds to the target head shape. The coordinate system may include a three-dimensional definition of the target head shape and can include different zones or quadrants. The attribute extractor 722 may compare portions of the scan data to corresponding zones or quadrants to identify which portions or areas of the scan data of the patient's head deviate from boundaries defined along the corresponding zones or quadrants in either direction (e.g., if the portions of the patient's head extend past or form a void relative to the boundaries defined along the corresponding zones or quadrants). In some embodiments, the attribute extractor 722 is configured to use threshold amounts to identify if the patient's head inappropriately deviates from the boundaries defined at the corresponding zones or quadrants of the target head shape. For example, if the patient's head deviates from the boundaries defined by the corresponding zones or quadrants of the target head shape within a threshold amount or percentage, then the geometry of the patient's head may be determined by the attribute extractor 722 as being acceptable and that these portions of the patient's head do not require correction. On the other hand, if the patient's head deviates from the boundaries defined by the corresponding zones or quadrants of the target head shape by at least the threshold amount or percentage, then the geometry of the patient's head may be determined by the attribute extractor 722 as requiring correction.

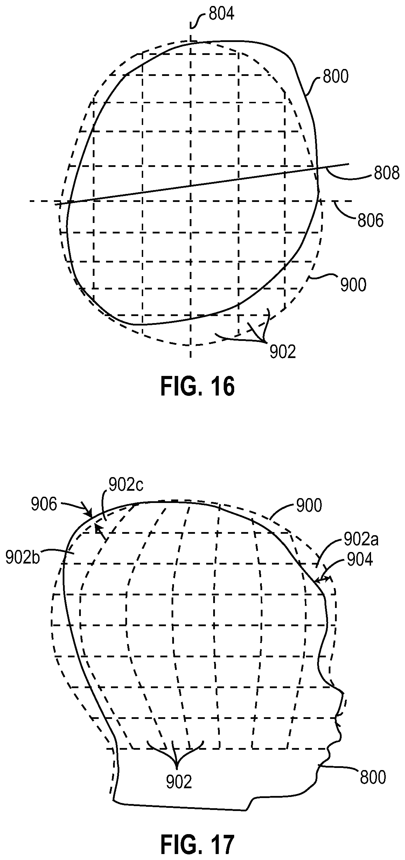

Referring to FIGS. 16 and 17, the attribute extractor 722 is configured to obtain the scan data, illustrated by scan data 800, and the target head shape, shown as target head shape 900. In some embodiments, the attribute extractor 722 is configured to compare the scan data 800 with the target head shape 900 and identify one or more zones or quadrants 902 within which the corresponding scan data 800 deviates. For example, as shown in FIG. 17, the scan data 800 deviates from a boundary defined at a first quadrant 902a of the target head shape 900 by a distance 904. Similarly, the scan data 800 deviates past a boundary defined at a second quadrant 902b and a third quadrant 902c by distance 906. It should be understood that "positive deviations" of the scan data 800 relative to the target head shape 900 indicate regions that require contact by pads due to the scan data 800 extending past the target head shape 900, while "negative deviations" of the scan data 800 relative to the target head shape 900 indicates regions that require voids due to the scan data 800 being within the target head shape 900. It should be understood that the quadrants 902 as shown herein are illustrative only and are not necessarily to scale. The quadrants 902 may have any shape or size, and the shape and size shown in FIGS. 16-17 should not be understood as limiting. In some embodiments, one or more quadrants 902 correspond to multiple arrays of holes 106 (e.g., multiple squares or 4-hole patterns of the holes 106).

As shown in FIGS. 16 and 17, the target head shape 900 may be divided into quadrants 902 which are used by the attribute extractor 722 in order to determine areas of the patient's head that have positive deviation, and therefore require contact by pads, and areas of the patient's head that have negative deviation and therefore require voids or spaces such that the patient's head may move or deform into the voids or spaces. In some embodiments, the corners of the quadrants 902 correspond to the holes 106 of the shell 200. In some embodiments, the attribute extractor 722 is configured to repeat the techniques described herein for different models of shells 200 that have different patterns of the holes 106, and therefore differently defined quadrants 902. In some embodiments, all the models of the shells 200 have a uniform or substantially similar pattern of holes 106 and therefore the attribute extractor 722 is configured to perform the techniques described herein once for a single pattern of quadrants 902.

Referring again to FIG. 15, the attribute extractor 722 is configured to provide one or more head attributes to the interpolator 724, according to some embodiments. The attribute extractor 722 may also provide the scan data to the interpolator 724. The interpolator 724 is also configured to receive the target head shape and the target attributes from the head shape database 732. In some embodiments, the interpolator 724 is configured to perform an interpolation technique in order to determine a shape for the helmet 100. For example, the interpolator 724 may receive identifications of which quadrants 902 have positive deviation of the patient's head, and which of the quadrants 902 have negative deviation of the patient's head. The interpolator 724 may determine a shape of inner surfaces of the pads 400 in order to achieve contact at one or more zones, regions, or quadrants of the patient's head, and one or more voids, spaces, etc., at regions where growth of the patient's head should be promoted. In some embodiments, the interpolator 724 is configured to combine the geometry of the desired head shape 900 and the scan data 800 of the patient's head shape in order to achieve the overall shape for the pads 400. For example, the interpolator 724 may compare the scan data 800 with the desired head shape 900 or use the comparisons from the attribute extractor 722 in order to determine a target shape that the pads 400 and the shell 200, when assembled, should form.

Referring to FIG. 15, the recommendation manager 710 includes a shell selector 712, a pad selector 714, an optimizer 716, an artificial intelligence (AI) 718, a custom pad manager 720, and a head shape change predictor 736, according to some embodiments. In some embodiments, the recommendation manager 710 is configured to receive one or more data inputs, including but not limited to, the scan data, the head attributes, the target head shape, one or more target attributes, interpolation data of the scan data and the desired head shape, etc. In some embodiments, the shell selector 712 is configured to select, based on the data inputs, a model or size of the shell 200 from the shell database 726. Similarly, the pad selector 714 is configured to select one or more pads from the pads database 728 and determine locations or placements within the selected shell. The optimizer 716 may be configured to implement an optimization-based decision making process for the shell selector 712 and/or the pad selector 714. The AI 718 may be configured to use the scan data and training data to predict a shell selection and corresponding pad selections and placement. In some embodiments, the custom pad manager 720 is configured to identify, based on the results of the pad selector 714, if one or more of the pads in the pads database 728 are not included in a kit. In some embodiments, the custom pad manager 720 is configured to determine if a custom pad is required and can prompt the manufacture of a custom pad having required parameters. The head shape change predictor 736 may be configured to predict a change of the patient's head over a treatment time to identify changes to the geometry of the patient's head. In some embodiments, the head shape change predictor 736 is configured to implement a model that is generated from a regression based on collected data over the treatment of a population of patients.

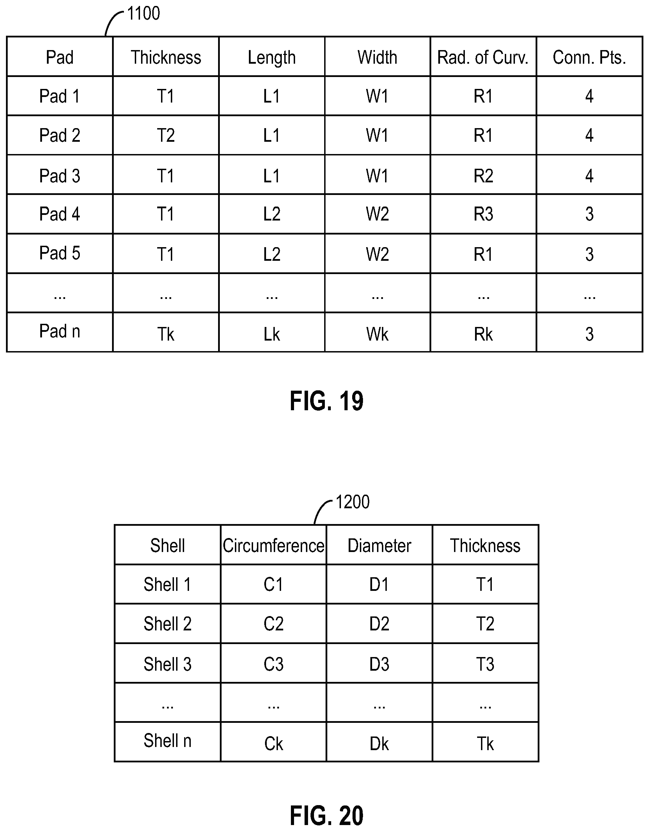

Referring to FIGS. 15 and 20, the shell selector 712 is configured to receive one or more shell options from the shell database 726 and select one of the shell options, according to some embodiments. In some embodiments, the shell options include different sizes such as small, medium, large, extra-large, etc. In some embodiments, the shell options are quantified by different parameters, illustrated by table 1200. As shown in table 1200, different shell models may have different attributes or dimensions such as different circumferences. The shell options may be quantified by circumference, diameter, and thickness, or any other parameter. In some embodiments, the shell selector 712 is configured to select the shell based on an overall size of the patient's head as identified by the attribute extractor 722 based on the scan data. For example, the shell selector 712 may use a predetermined set of rules to select a corresponding shell from the shell database 726 based on diameter, circumference, volume, etc., of the patient's head. In some embodiments, the shell selector 712 is configured to use an output of the head shape change predictor 736 to select the shell from the shell database 726. For example, the head shape change predictor 736 may predict, based on a required amount of therapy time, a growth that is predicted to occur in the patient's head (e.g., over the course of weeks or months). In some embodiments, the growth or change in the diameter, volume, circumference, etc., of the patient's head is used by the shell selector 712 to select a shell from the shell database 726 that is usable over a course of the patient's therapy. For example, if the patient's head is predicted to grow a significant amount, the shell selector 712 may select a size of shell that can accommodate pads and appropriate therapy for the patient with sufficient space over a course of the patient's therapy. In some embodiments, the shell selector 712 is configured to provide an identification of the selected shell to the pad selector 714 for use in selecting appropriate pads from the pads database 728. The shell selector 712 may use a predetermined set of rules to select the shell from the shell database 726 based on the size or attributes of the patients head as indicated in the scan data 800, and/or as predicted by the head shape change predictor 736.

Referring particularly to FIGS. 15 and 19, the pad selector 714 is configured to receive one or more pads options from the pads database 728 and select pads for installation at particular locations in the shell 200, according to some embodiments. In some embodiments, the pads database 728 stores information regarding a geometry, such as a thickness, length, width, radius of curvature, connection points, etc., of each of one or more available pads. For example, the pads database 728 may generally include information for each of a plurality of different models (e.g., Pad 1, Pad 2, Pad 3, Pad n, etc., as shown in column 1 of table 1100), regarding the size and shape of the plurality of different models of pads. In some embodiments, the pad selector 714 is configured to select, for the quadrants 902, an appropriate model of pad based on the scan data 800 and the target head shape 900. For example, the pad selector 714 may perform an algorithm in which the pad selector 714 selects pads for a corresponding shell (e.g., the shell selected by the shell selector 712). In some embodiments, the pad selector 714 is configured to select pads for quadrants at which the scan data 800 has positive deviation relative to the target head shape 900 such that the selected pads will contact the patient's head along the quadrants 902 where the scan data 800 positively deviates from the target head shape 900. In some embodiments, the pad selector 714 is configured to select the model of pads for each of, or groups of, the quadrants 902 based on an indication of if the scan data 800 has positive or negative deviation relative to the target head shape 900, as well as a degree of deviation (e.g., distance 906 or distance 904). In some embodiments, for quadrants or zones where the scan data 800 has negative deviation relative to the target head shape 900 (e.g., regions of the patient's head that are recessed, caved in, etc.), the pad selector 714 may select pads such that a surface of the pads terminates at the target head shape 900.

Referring still to FIGS. 15 and 19, the pad selector 714 may use one or more of the shell models (e.g., Shell 1, Shell 2, Shell 3, Shell n, etc., as shown in FIG. 20) to select different combinations of pads from the pads database 728 corresponding to each potential shell. In some embodiments, the pad selector 714 is configured to use known geometry of the shells in the shell database 726 to estimate or project a location of an inner surface of the shells. In some embodiments, the pad selector 714 is configured to, based on the estimated or projected location of the inner surface of the shells, the direction of deviation of the scan data 800 relative to the target head shape 900 (e.g., positive or negative), the degree of deviation of the scan data 800 relative to the target head shape 900 (e.g., distance 904, distance 906, etc.), and the boundary of the target head shape 900, select appropriate pads for each of the different shells such that the inner surfaces of the pads contacts or abuts the patient's head in desired locations or leaves spaces in other desired locations. For example, the pad selector 714 may use the scan data 800 and the target head shape 900 to "add" the scan data 800 and the target head shape 900 to produce a combined shape. The combined shape may indicate boundaries at which the pads selected by the pad selector 714 should terminate. The pad selector 714 may determine distances between an inner surface of each of the selectable shells, and outer surfaces or boundaries of the combined shape, and select pads for each quadrant to substantially "fill" those areas. In this way, the pad selector 714 may select pads having appropriate thickness, size, shape, radius of curvature, etc., such that the pads contact the patient's head at quadrants of positive deviation, and leave room for the patient's head at quadrants of negative deviation (without allowing the patient's head to be reformed beyond the target head shape 900 at locations of negative deviation). In some embodiments, the pad selector 714 is configured to perform the functionality of selecting the pads for the shell that is selected from the shell selector 712 after the shell selector 712 performs its selection. In some embodiments, the pad selector 714 is configured to perform the functionality of selecting the pads for all of the shells of the shell database 726 since different shells may have different thicknesses or sizes, and therefore have differently positioned inner surfaces requiring the selection of pads having different thicknesses.

Referring to FIG. 15, the shell selector 712 and the pad selector 714 may perform their functionality in parallel, or one may perform its functionality based on the outputs of the other. For example, the pad selector 714 may first select pads for quadrants where the scan data 800 indicates positive deviation of the patient's head beyond the target head shape 900 based on the degree of deviation at the quadrants (e.g., either positive or negative deviation). After the pad selector 714 has selected appropriate thickness of pads based on the degree of deviation at the quadrants, the pad selector 714 may provide the selected pads to the shell selector 712 for use in selecting a shell that can accommodate the selected pads and placement thereof. Likewise, the shell selector 712 may initially select a shell based on the scan data 800 and provide the selected shell to the pad selector 714 for use in selecting pads and pad placement in the selected shell.

Referring to FIG. 15, the optimizer 716 of the recommendation manager 710 may be configured to use a predictive model to quantify a metric one or more possible combinations of shells and pads as identified by the shell selector 712 and the pad selector 714. The optimizer 716 may quantify how closely the selected shells and pad combinations as provided by the shell selector 712 and the pad selector 714 achieve a desired boundary (e.g., a quantified error between a target inner surface location to be defined by the shell and the pads, and an actual inner surface location defined by the shell and the pads). The optimizer 716 may construct and perform an optimization problem to select one of the available shells and pad combinations as provided by the shell selector 712 and the pad selector 714 that optimizes (e.g., maximizes, minimizes, etc.) or otherwise is predicted to achieve a best result in terms of the quantified metric. In some embodiments, the optimizer 716 is configured to use a model to predict healing or reshaping progress of the patient's head as the metric. In some embodiments, the optimizer 716 is configured to predict a likelihood that one or more pads will need to be ground down or manually reshaped by treatment personnel, and select a combination of the shell and pads out of the combinations identified by the shell selector 712 and the pad selector 714 that achieves the least predicted manual reshaping. In some embodiments, the optimizer 716 is configured to use the outputs of the head shape change predictor 736, and select the combination of shell and pads that can be worn for an entirety of or a longest amount of an estimated therapy time for the patient. For example, if the optimizer 716 identifies that a first combination of the shell and pads as provided by the shell selector 712 and the pad selector 714 is predicted to be able to be worn by the patient over an entirety of the therapy time or head-reshaping time period, the optimizer 716 may select the first combination as the optimal combination for use.

The AI 718 may be a neural network, a deep learning network, a convolutional neural network, etc., that is trained on both scan data for a variety of patients, selected shells or pads and corresponding data (e.g., size, shape, thickness, radius of curvature, etc.), as well as corresponding reshaping progress. In some embodiments, the AI 718 is trained based on aggregate training data of the selected shells or pads and corresponding data, the scan data, and the reshaping progress for a population of patients. The AI 718 may be trained to select both the shell and the pads, as well as placement of the pads within the shell based on the scan data in order to achieve an optimal reshaping progress (e.g., least amount of time, maximum comfort for the patient, best results of the reshaping process, or any combination thereof). In some embodiments, the AI 718 is trained based on training data to identify types of head deformation or conditions of the patient based on the scan data 800 such as brachycephaly, plagiocephaly, scaphocephaly, etc. The AI 718 may provide recommended shell and pad data to the output manager 730 for display on display device 616. In some embodiments, the AI 718 is configured to perform the functionality of the head shape change predictor 736 either in terms of growth of the patient's head, or in terms of reshaping changes in order to predict an impact that a selected combination of shell, pads, and pad placement will have on the patient's head over time.

In some embodiments, the pad selector 714 may identify that the patient requires a pad having specific geometry, thickness, size, shape, radius of curvature, etc., that is not available in the pads database 728. In some embodiments, the pads database 728 is a standardized database that includes pads having predetermined size, shape, thickness, radius of curvature, etc., for common treatment plans. In certain cases, however, a custom pad may be required to accommodate specific geometry of the patient's head. If the pad selector 714 identifies that none of the available options in the pads database 728 can be used for one or more quadrants, the pad selector 714 can provide an indication to the custom pad manager 720 including the corresponding scan data 800, the target head shape 900, indicated quadrants that require custom pads, etc. The custom pad manager 720 may use the scan data 800, the target head shape 900, and the indicated quadrants in order to determine one or more characteristics for a custom pad. In some embodiments, the requirements for the custom pad can include thickness, length, width, radius of curvature, number of required connection points, geometry, etc. The custom pad manager 720 is configured to provide the requirements for the custom pad to the output manager 730 in order to initiate a manufacturing process of the custom pad. The pad selector 714 may identify that a custom pad is required in response to determining that none of the pad options in the pads database 728 when placed at one or more quadrants, terminate at a desired location (e.g., to abut the patient's head at areas of positive deviation).

The head shape change predictor 736 may implement one or more predictive models in order to predict growth of the patient's head over time. In some embodiments, the head shape change predictor 736 uses a model that is trained on known growth progression of a patient's head over time. In some embodiments, the head shape change predictor 736 is configured to use the scan data 800 or attributes extracted from the scan data 800 (e.g., volume of the patient's head, etc.) as an input to the one or more predictive models. The head shape change predictor 736 may estimate an overall change in the patient's head, as well as the corresponding change in the target head shape 900 which may be provided to the shell selector 712, the pad selector 714, the optimizer 716, the AI 718, etc., for use in selecting appropriate shells and pads for the patient.

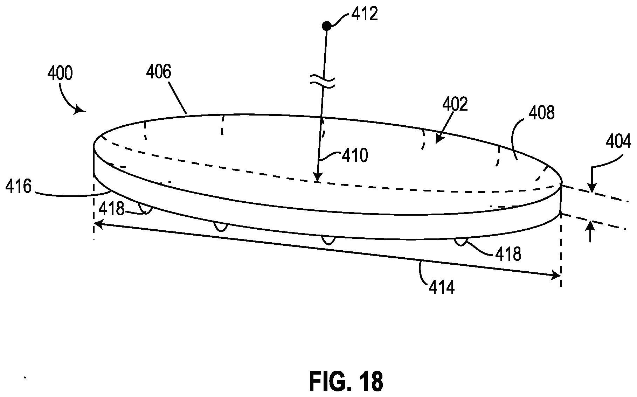

Referring to FIG. 18, another one of the pads 400 is shown including a curved inner surface 408 (e.g., a concave inner surface). In some embodiments, an outer surface 416 may have a curved or convex shape corresponding to the curved inner surface 408 or corresponding to geometry of the shell 200. The pad 400 has a length 414 and thickness 404. The pad 400 also has a radius of curvature 410 which indicates curvature of the curved inner surface 408. In some embodiments, the pad 400 also includes one or more connection points 418 (e.g., protrusions, clips, interlocking members, etc.). The radius of curvature 410, the thickness 404, the length 414, the number of connection points 418, a width of the pad 400, as well as an overall shape or footprint of the pad 400 may all be quantifiable decision variables that are stored in the pads database 728 and used to select appropriate pads and placement by the pad selector 714.

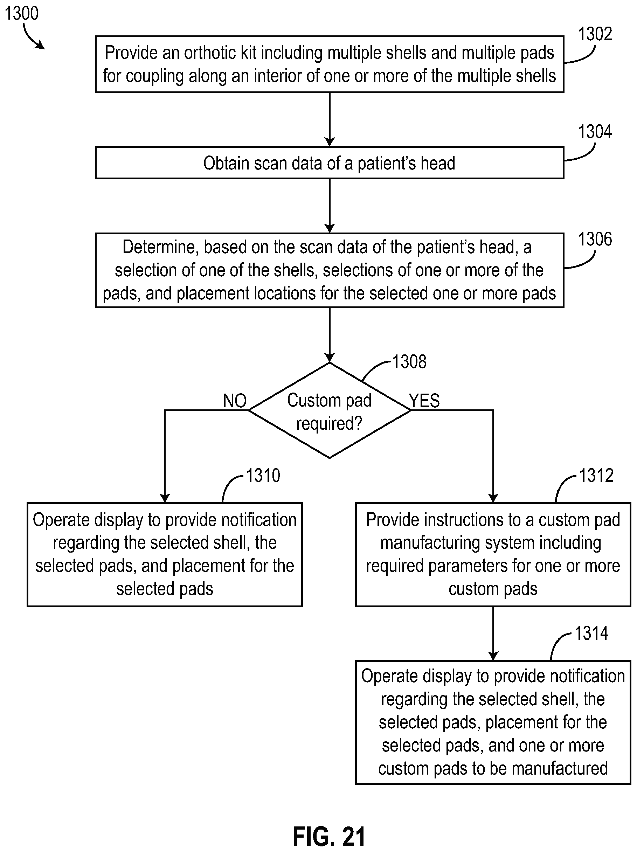

Referring to FIG. 21, a flow diagram of a process 1300 for selecting a shell and pads for a pediatric orthotic device includes steps 1302-1314, according to some embodiments. In some embodiments, the process 1300 is performed by the recommendation system 700 using any of the techniques described in greater detail above with reference to FIGS. 15-20. The process 1300 can be performed in order to select a shell from multiple shells in a kit, as well as pads from multiple pads in the kit. The process 1300 can also be performed in order to determine a placement of the pads within the selected shell. In some embodiments, the process 1300 is performed in order to identify if a custom pad is required for a particular patient, and to prompt manufacturing of the custom pad.

The process 1300 includes providing an orthotic kit including multiple shells and multiple pads for coupling along an interior of one or more of the multiple shells (step 1302), according to some embodiments. In some embodiments, the shells each include a plurality of openings, holes, or connection points disposed in at least two arrays about the shells. In some embodiments, the multiple pads are universal or standardized pads that can each be installed into any of the multiple shells. The multiple shells may be different in overall size such as small, medium, large, etc. In some embodiments, the pads are configured for most typical or common application of pediatric orthotic devices. For example, the size, shape, thickness, radius of curvature, etc., of the pads may be for the most common types of conditions or head deformations that are present in a population of children or infants. In some embodiments, the orthotic kit is provided including the multiple pads and multiple shells such that a clinician can assemble one of the shells in combination with one or more of the pads to provide cranial remodeling therapy to a patient.

The process 1300 includes obtaining scan data of a patient's head (step 1304), according to some embodiments. In some embodiments, the scan data is obtained by scan device 612. Step 1304 may be performed any time the patient arrives for fitting or adjustment of a pediatric orthotic device, or at regular intervals. Step 1304 may include obtaining the scan data at a recommendation tool such as recommendation tool 702, or any other computer system. The scan data may indicate areas of the patient's head (e.g., quadrants) at which positive deviation of the patient's head occurs and other areas of the patient's head (e.g., other quadrants) at which negative deviation of the patient's head occurs.

The process 1300 includes determining, based on the scan data of the patient's head, a selection of one of the shells, selections of the one or more pads, and placement locations for the selected one or more pads (step 1306), according to some embodiments. In some embodiments, step 1306 is performed based on an interpolation or comparison between the scan data of the patient's head and an ideal or target head shape. In some embodiments, step 1306 includes identifying one or more quadrants of the scan data at which positive deviation or negative deviation of the scan data occurs. Positive deviation may indicate locations at which the patient's head protrudes beyond the ideal or target head shape, while negative deviation indicates locations at which a space is formed between the patient's head and the ideal or target head shape. Step 1306 can be performed based on the interpolation between the scan data of the patient's head and the ideal or target head shape. In some embodiments, step 1306 is performed by using an algorithm or a set of steps to identify a degree of positive or negative deviation at each quadrant, identify a curvature of the patient's head at the quadrants, and select a corresponding pad having appropriate thickness, curvature, and size. In some embodiments, step 1306 is performed by using machine learning, artificial intelligence, or neural network. In some embodiments, step 1306 is performed using an optimization process or technique.

Step 1306 may also be performed by accounting for predicted growth of the patient's head. In some embodiments, the shell is selected based on the predicted growth of the patient's head over an expected course of duration that the pediatric orthotic will be worn such that the patient does not need to switch shells partially through the duration. In some embodiments, the shell and the pads are selected concurrently or simultaneously. In some embodiments, the pads are selected based at least partially on dimensions of the shell that is selected. In some embodiments, the shell is selected at least partially based on which pads are selected. In some embodiments, step 1306 is performed by the processing circuitry 704 of the recommendation tool 702. In some embodiments, step 1306 is performed by selecting the shell from a plurality of pre-manufactured shell options that are provided in the orthotic kit. In some embodiments, step 1306 is performed by selecting the pads from a plurality of pre-manufactured pad options that are provided in the orthotic kit.

The process 1300 includes determining if a custom pad is required (step 1308), according to some embodiments. In some embodiments, step 1308 is performed in response to step 1306. For example, during step 1306 while determining the selection of the shells and the selections of the pads, the recommendation tool 702 may identify that none of the available pad options sufficiently meet the requirements given the patient's head shape. In response to determining that a pad is required that is not available in the orthotic kit (step 1308, "YES"), process 1300 proceeds to step 1312. If the pads provided in the orthotic kit are sufficient to provide therapy at all the locations about the patient's head (step 1308, "NO"), process 1300 proceeds to step 1310.

The process 1300 includes operating a display to provide a notification regarding the selected shell, the selected pads, and placement for the selected pads within the selected shell (step 1310), according to some embodiments. In some embodiments, step 1310 is performed by the recommendation tool 702 and the display device 616. In some embodiments, step 1310 includes providing instructions to a clinician regarding which of the shells and pads to use for a particular patient. The instructions may include an indication of which of the multiple shells to use, and which pads to place in which locations on the shell (e.g., which quadrants the pads should span across). In some embodiments, step 1310 is performed in response to no custom pads being required (step 1308, "NO").

The process 1300 includes providing instruction to a custom pad manufacturing system including required parameters for one or more custom pads (step 1312), according to some embodiments. In some embodiments, step 1312 is performed by uploading instructions to a remote or cloud computing system at a manufacturing facility. In some embodiments, step 1312 is performed by providing the instructions to an on-site additive manufacturing system (e.g., the 3d printer 614). Step 1312 may be performed by providing the scan data of a corresponding area or quadrant(s) of the patient's head at which the recommendation tool 702 has determined that none of the available pads in the orthotic kit can properly be used. In some embodiments, step 1312 is performed in order to produce pads for patients that have cranial deformations that are rare.

The process 1300 includes operating the display to provide a notification regarding the selected shell, the selected pads, placement for the selected pads, and one or more custom pads to be manufactured (step 1314), according to some embodiments. In some embodiments, step 1314 is performed similarly to step 1310 but includes an additional notification regarding the custom pads that are to be manufactured by the pad manufacturing system. In this way, the clinician is notified regarding which pads can be installed immediately, and which pads require manufacturing.

### Configuration of Exemplary Embodiments

As utilized herein, the terms "approximately", "about", "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the invention as recited in the appended claims.

It should be noted that the terms "exemplary" and "example" as used herein to describe various embodiments are intended to indicate that such embodiments are possible examples, representations, and/or illustrations of possible embodiments (and such term is not intended to connote that such embodiments are necessarily extraordinary or superlative examples).

The terms "coupled," "connected," and the like, as used herein, mean the joining of two members directly or indirectly to one another. Such joining may be stationary (e.g., permanent, etc.) or moveable (e.g., removable, releasable, etc.). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," "between," etc.) are merely used to describe the orientation of various elements in the figures. It should be noted that the orientation of various elements may differ according to other exemplary embodiments, and that such variations are intended to be encompassed by the present disclosure.

The hardware and data processing components used to implement the various processes, operations, illustrative logics, logical blocks, modules and circuits described in connection with the embodiments disclosed herein may be implemented or performed with a general purpose single- or multi-chip processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein. A general purpose processor may be a microprocessor, or, any conventional processor, controller, microcontroller, or state machine. A processor also may be implemented as a combination of computing devices, such as a combination of a DSP and a microprocessor, a plurality of microprocessors, one or more microprocessors in conjunction with a DSP core, or any other such configuration. In some embodiments, particular processes and methods may be performed by circuitry that is specific to a given function. The memory (e.g., memory, memory unit, storage device, etc.) may include one or more devices (e.g., RAM, ROM, Flash memory, hard disk storage, etc.) for storing data and/or computer code for completing or facilitating the various processes, layers and modules described in the present disclosure. The memory may be or include volatile memory or non-volatile memory, and may include database components, object code components, script components, or any other type of information structure for supporting the various activities and information structures described in the present disclosure. According to an exemplary embodiment, the memory is communicably connected to the processor via a processing circuit and includes computer code for executing (e.g., by the processing circuit and/or the processor) the one or more processes described herein. References to "a" or "the" processor should be understood to encompass a plurality of processors individually or collectively configured to carry out operations as described herein.

The present disclosure contemplates methods, systems and program products on any machine-readable media for accomplishing various operations. The embodiments of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. Combinations of the above are also included within the scope of machine-readable media. Machine-executable instructions include, for example, instructions and data which cause a general purpose computer, special purpose computer, or special purpose processing machines to perform a certain function or group of functions.

Although the figures and description may illustrate a specific order of method steps, the order of such steps may differ from what is depicted and described, unless specified differently above. Also, two or more steps may be performed concurrently or with partial concurrence, unless specified differently above. Such variation may depend, for example, on the software and hardware systems chosen and on designer choice. All such variations are within the scope of the disclosure. Likewise, software implementations of the described methods could be accomplished with standard programming techniques with rule-based logic and other logic to accomplish the various connection steps, processing steps, comparison steps, and decision steps. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y, and Z," unless specifically stated otherwise, is otherwise understood with the context as used in general to convey that an item, term, etc. may be either X, Y, Z, X and Y, X and Z, Y and Z, or X, Y, and Z (i.e., any combination of X, Y, and Z). Thus, such conjunctive language is not generally intended to imply that certain embodiments require at least one of X, at least one of Y, and at least one of Z to each be present, unless otherwise indicated.

It is important to note that the construction and arrangement of the systems as shown in the exemplary embodiments is illustrative only. Although only a few embodiments of the present disclosure have been described in detail, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts or elements. It should be noted that the elements and/or assemblies of the components described herein may be constructed from any of a wide variety of materials that provide sufficient strength or durability, in any of a wide variety of colors, textures, and combinations. Accordingly, all such modifications are intended to be included within the scope of the present inventions. Other substitutions, modifications, changes, and omissions may be made in the design, operating conditions, and arrangement of the preferred and other exemplary embodiments without departing from scope of the present disclosure or from the spirit of the appended claim.

## Claims

1. A method **characterized by**:
obtaining scan data of a patient's head;
using computer assisted design to generate a print file of a cranial remodeling orthosis (CRO) based on the scan data, wherein the CRO comprises at least one integrated closure mechanism; and
performing additive manufacturing by a 3D printer to produce the CRO using the print file of the CRO.

2. The method of Claim 1, further **characterized in that** the CRO comprises an outer shell configured to receive a patient interfacing insert, wherein at least one of the outer shell or the patient interfacing insert are manufactured by the 3D printer.

3. The method of Claim 2, further **characterized in that** the outer shell comprises a first section and a second section.

4. The method of Claim 3, further **characterized in that** the at least one integrated closure mechanism comprises a hinge.

5. The method of Claim 4, further **characterized in that** the hinge is configured to facilitate relative rotation of the first section and the second section to transition the CRO between an open configuration and a closed configuration.

6. The method of Claim 1, 2, 3, or 4, further **characterized in that** using the computer assisted design to generate the print file comprises modifying a position, thickness, and trim area of the print file such that the CRO produced using the print file is configured to provide therapy to reshape the patient's head over time.

7. The method of Claim 1, 2, 3, or 4, further **characterized in that** the CRO is a helmet configured to be worn on the patient's head.

8. A system for manufacturing a cranial remodeling orthosis (CRO), the system **characterized by**:
a scan device configured to obtain scan data of a patient's head;
processing circuitry configured to:
obtain the scan data of the patient's head; and
use computer assisted design to generate a print file of the CRO based on the scan data, wherein the CRO comprises at least one integrated closure mechanism; and
a 3D printer configured to perform additive manufacturing to produce the CRO using the print file of the CRO.

9. The system of Claim 8, further **characterized in that** the CRO comprises an outer shell configured to receive a patient interfacing insert, wherein at least one of the outer shell or the patient interfacing insert are manufactured by the 3D printer.

10. The system of Claim 8 or 9, further **characterized in that** the outer shell comprises a first section and a second section or further **characterized in that** the at least one integrated closure mechanism comprises a hinge.

11. A cranial remodeling orthosis (CRO) configured to be worn on a patient's head, the CRO **characterized by**:
an outer shell comprising at least one integrated closure mechanism; and
internal padding configured to couple with the outer shell along an inwards facing surface of the outer shell,
wherein the CRO is configured to adjust a shape of a skull of the patient over time to increase symmetry of the patient's head, and
wherein at least one of the outer shell or the internal padding are manufactured by additive manufacturing.

12. The CRO of Claim 11, further **characterized in that** the outer shell comprises a first section and a second section, wherein the at least one integrated closure mechanism comprises a hinge, wherein the hinge is configured to facilitate relative rotation of the first section and the second section to transition the CRO between an open configuration and a closed configuration.
